# EUROPEAN PATENT SPECIFICATION

(11) **EP 1 289 530 B1**
(45) Date of publication and mention of the grant of the patent: **04.10.2006**
(21) Application number: 01938401.5
(22) Date of filing: 13.06.2001
(51) Int. Cl.: A61K 31/66, A61P 37/02

(54) **LIPIDS FOR MODULATING IMMUNE RESPONSE**
LIPIDE ZUR ÄNDERUNG DES ABWEHRSYSTEMS
LIPIDES DESTINES A MODULER UNE REPOSE IMMUNE

(30) Priority: 14.06.2000 GB 0014437; 01.11.2000 GB 0026667; 20.11.2000 GB 0028239
(43) Date of publication of application: 12.03.2003
(73) Proprietor: PORTER, William Leslie, Diss, Norfolk IP22 1RY (GB)
(72) Inventor: PORTER, William Leslie, Diss, Norfolk IP22 1RY (GB)
(74) Representative: Lipscombe, Martin John
(86) International application number: PCT/GB2001/002568
(87) International publication number: WO 2001/095914

(56) References cited:
- WO-A-01/49277
- WO-A-99/03479
- US-A- 4 978 654
- US-A- 5 434 183
- PATENT ABSTRACTS OF JAPAN vol. 006, no. 250, 9 December 1982 (1982-12-09) & JP 57 145810 A (TOYAMA KAGAKU KOGYO KK), 9 September 1982 (1982-09-09)

## Description

This invention relates generally to immune response modulation in man and animals and more especially to lipids and/or lipid-like preparations to cause or modulate immune response, both systemic and mucosal, preferably but not essentially effective by transmucosal administration. More particularly, the present invention concerns the use of lipid and lipid-like substances, of non-bacterial origin, in the preparation of a composition to enhance or modulate the mucosal and systemic immune response to antigenic challenge, as a means of preventing and treating infectious and immune disease.

### Background to the invention

Control of infectious diseases in man and animals is a subject of major interest and research, since existing means of control have known deficiencies. Thus, antibiotics are continually rendered ineffective by the development of resistant strains of microorganisms.

An opportunity therefore exists for the broad-spectrum enhancement of the natural immune response to infectious agents, as a means of treating and preventing a wide range of diseases. Since procedures involving parenteral injections can entail a risk of injection-site infections, particularly in third world situations, it would be preferable, although not exclusively so, that any such measure should be effective by the oral route.

It has been postulated that certain killed bacteria, and lipid extracts thereof incorporated in liposomes, given orally to animals, can enhance immune response to certain protein antigens. Antigenicity of certain bacterial lipids, given parenterally, is known, and an ability to cause immunomodulation has been reported for a few lipid-like chemical structures, given parenterally.

Enhancement of growth rate in young animals and the reduction of incidence of diarrhoea in animals orally receiving killed bacterial preparations, has also been reported, and it has been reported that enhancement of growth rate is achievable in animals receiving lipid-containing extracts of such bacterial preparations adsorbed onto non-lipid particulate matter, as well as in animals receiving similarly administered preparations of whole killed bacterial. It has also been shown that a chloroform/methanol-derived lipid extract of bacteria, given by mouth in liposomes, can enhance the cellular and antibody response to subsequent immune challenge.

It is also known that protein antigenic substances gain access from the gut to the reticulo-endothelial system via lymphoid tissue in the wall of the intestine, for instance Peyer's patches. Further it is known that such materials gain access more readily if they are incorporated in liposomes, bilosomes or similar lipid-containing vesicles or in beads formed by, for instance, polyethylene glycol.

In general, there is little knowledge of the identity of lipids which may modulate immune response in animals and humans, or the conditions necessary to render administration of lipids effective for the purpose, especially in the case or oral administration.

It is now known that there may be theoretical advantages in antigens and adjuvants gaining access to the immune system via mucosal surfaces, rather than by parenteral administration, in that access to the immune system may be facilitated.

Also, access via the mucosa, as by the gastro-enteric route, gives the possibility of modulating mucosal, as well as systemic, immune response.

A principal aim of the invention is to facilitate access to the body's immune system of certain non-bacterial lipids, preferably although not exclusively administered transmucosally, particularly orally, whereby to stimulate immunity and/or to modulate the immune response to antigenic stimulus. A means is also shown whereby the immune response to antigens may be suppressed, where it may be desirable to do so, as in the prevention and treatment of immune disease.

It is shown that lipids having particular physio-chemical characteristics are best suited for the purposes of this invention.

### Principal features of the Invention

According to one aspect of the invention there is provided use of a synthetic lipid or lipid from animal or vegetable oil in accordance with claim 1.

For the purpose of this application, lipids are defined as esters of glycerol (propane 1,2,3-triol) with fatty acids, also including 1,2-di-*O*-acylglycerol joined at oxygen 3 by a glycosidic linkage to a carbohydrate, also derivatives of glycerol in which one hydroxy group, commonly but not necessarily primary, is esterified with fatty acids, thus excluding those organic compounds consisting of a trisaccharide repeating unit with oligosaccharide side chains and acyl or amine linked fatty acids.

The complete product may comprise any one or a mixture of one or more phospho-, neutral or glyco lipids, which may be synthesised, or may be contained in or derived from natural materials such as animal or vegetable oils from which they may or may not be extracted or concentrated as by distillation, fractionation, phoresis or other known processes.

The lipids are as specified in a) to f) below, given to man or animals in non-particulate or particulate form as specified below, by parenteral or transmucosal administration, for the purpose of modulating the immune response to antigens.

The lipid/s present in the composition of the present invention is characterised by the following:
a) There should be at least one alkyl or acyl chain.
b) The chain length should be 10 to 22 carbon atoms, preferably 12 to 20 and more preferably 16 to 20 or even 18 to 20.
c) Where a lipid has a head group, the head should preferably have a mass of at least 269 Atomic Mass Units (AMU) and a volume of at least 167 cm⁻³mol⁻¹.
d) Where a lipid has a head group, it may have a combined partial electrical charge of - 1.015eV or more strongly negative, but preferably of -1.044eV or more strongly negative.
e) Where the head group has a charge, it may be in a single centre, but preferably should be in multiple centres.
f) The head group charge to volume ratio is preferably -0.004eV/cm⁻³mol⁻¹ or more strongly negative, and more preferably -0.006eV/cm⁻³mol⁻¹ or more strongly negative.

Whilst it might be postulated that lipids conforming to one or more of the above physicochemical criteria might be particularly useful in facilitating linkage between antigens and receptor mechanisms of the immune system, the invention is by no means confined to this theory.

### Further features

Lipids may be administered trans-mucosally, or they may be administered parenterally. When given trans-mucosally, especially orally, for the purpose of enhancing immune response, it is desirable though not essential, that the lipids be given in particulate form.

That the particle size is within the predetermined limits may be established by known methods, e.g. microscopic examination or by sizing techniques employing beams of radiation such as laser beams, and by readily available sizing instruments such as the Malvern Mastersizer.

Lipids as specified in a) to f) above, consisting of or obtained from natural materials by means such as extraction with solvents such as chloroform and methanol, or alternatively of synthetic origin, may be rendered into a particulate form by incorporation into or onto liposomes or bilosomes or other appropriately sized particulate matter, such as silica, titanium dioxide, or kaolin particles, or other such material capable of absorbing or adsorbing lipids.

This may be achieved, for instance, by rendering the lipid or lipids into a liquid of low viscosity, as by heating, dissolving in solvents such as chloroform or methanol, or by forming into an aqueous preparation by means of an emulsifying agent, to form a emulsion or colloid.

The particles to be coated are then dispersed in the liquid by stirring, shaking vibration, sonication or other known means of dispersion. The liquid can then be removed to leave a coating of the lipid or lipid-like substance on the particle surfaces. This may be achieved by, for instance, evaporation of solvents or aqueous medium, centrifugation (batch or continuous flow) or filtration, preferably cross-flow filtration. When the lipid can be shown, for instance by microscopic examination, to be applied to the particles, a carrier may be added in suitable volume to provide a convenient dosage form.

It is preferable, though not essential that, where the lipid or lipids, (particularly if single chain, neutral or unsaturated) are administered in liposomes, bilosomes or other vesicular form, or as a suspension, protein or peptide antigens should not be directly mixed or combined with the lipid, or combined within vesicles. However, such antigens may be given concurrently with the lipid or lipids, in the same formulated dose, if not so directly combined. Alternatively, they may be given by separate administration, as in modulation of the immune response to antigenic challenge resulting from infection or other cause.

The non-bacterial lipid, whether or not incorporated into liposomes, bilosomes or attached to suitably sized particles, may be incorporated into particulate formulations of wet or dry powder or granular mixes. Alternatively, they may be incorporated into liquids such as water, oil, milk or any beverage. Powder formulation may also be given in tablets, capsules or paste, or may be incorporated into foods.

In the case of carrier particles, these should preferably have a size range of 0.1 to 100 microns, preferably 0.1 to 25 microns and most preferably 0.2 to 15 or even 0.3 to 10 microns, and ideally 0.4 to 8 microns or 0.5 to 5 microns.

One feature of the invention thus provides a preparation of lipids, as specified in a) to f) above, which may be administered parenterally or trans-mucosally in particulate form, with particles of dimensions within the above stated limits, or in non-particulate form.

The lipids as specified above may be phospholipids, glycolipids or neutral lipids, saturated or unsaturated, with or without branching in their fatty acid chains, within the categories of:-
Phosphatidic acids
Phosphatidylethanolamines
Phosphatidylserines
Phosphatidylinositols
Phosphatidylcholines
Cardiolipins
Triglycerides
1,2-Diglycerides
1,3-Diglycerides
Monoglycerides
and their isomers

More particularly, they may include:-
Phosphatidylcholine, dioleoyl
Trilinolenin
L-*a*-Phosphatidic acid
L-*a*-Phosphatidicacid, dimyristoyl
L-*a*-Phosphatidylcholine, dimyristoyl
L-*a*-Phosphatidylcholine, dipentadecanoyl
L-*a*-Phosphatidylcholine, diheptadecanoyl
DL-*a*-Phosphatidylchaline, diheptadecanoyl
L-*a*-Phosphatidylethanolamine, distearoyl
DL-*a*-Phosphatidylethanolamine, dimyristoyl
1,2-Distearoyl-sn-glycero-3-phosphoethanolamine, dipalmitoyl
L-*a*-Phosphatidylethanolamine
DL-*a*-Phosphatidyl-L-serine, diheptadeanoyl

The above are referred to as Group 1 Lipids, in Example 4, below.
N-Palmitoyl digydroglucocerebroside
Trymyristin
Tripentadecanoin
Tripalmitin
1,2 Diplamitoyl-3-myristoyl-rac-glycerol
1,2 Distearloyl-3-myristoyl-rac-glycerol
1,2 Distearoyl-3-palmitoyl-rac-glycerol
rac-1,2-Dimyristoyl-3-palmitoylglycerol

The above are referred to as Group 2 Lipids, in Example 4, below.

Such lipids may be given trans-mucosally, in particulate form as specified above, or they may be given parenterally, in particulate or non-particulate form. Trans-mucosal administration may be, for instance, by the oral route, by buccal or sub-lingual placement or spray, or by intranasal spray.

They may be given together with the antigen or antigens, as an adjuvant, or they may be given separately, especially prior to the antigenic challenge, in order enhance the immune response.

For some purposes of immune modulation, such as where the intention is to suppress rather than enhance the immune response to antigenic stimulus, for instance, in the control of immune diseases, the lipid materials may be given by a trans-mucosal route, but in non-particulate form. For these purposes, they may be administered dispersed in liquid emulsions, colloids, suspensions, solutions or other liquid dispersions or in gels, pastes or in dispersible in solid forms incorporating e.g. starch or cellulose, or may be administered in aerosol sprays.

The immunologically active material may be formed into liposomes by known techniques, or mixed with oily or viscous preparations to form liposomes, or may be otherwise incorporated into or onto carrier particles of appropriately sized particulate material, such as colloidal silica, silicilic acid, kaolin particles or other such material capable of absorbing or adsorbing lipids. Such particles may be incorporated into powder, tablets, capsules, food, liquids or beverages. The sizes of such liposomes or carrier particles incorporating the immunologically active material may have a size range of 0.1 to 100 microns, preferably 0.2 to 25 microns, most preferably 0.2 to 15 or even 0.3 to 10 microns, and ideally 0.4 to 8 microns or 0.5 to 5 microns.

Verification examples are now described.

### Example 1 (outside the scope of the present invention)

Comparison is made between two tests of immune globulin production in mice, following oral administration of a test antigen, where the mice received daily doses of partially autolysed and autoclaved *Bacillus subtilis.*

In test A, *Bacillus subtilis* was grown in a clear liquid medium containing yeast extract, sucrose, magnesium and other soluble nutrients, according to well-established procedures. The bacterial cells were separated from the culture medium by centrifugation and allowed to stand for 24 hours at room temperature. The cells were examined grossly and microscopically at intervals of about 6 hours. After this period, and before any visible physical breakdown of the bacteria was observed, the cells were autoclaved at 115 degrees C for 20 minutes. Microscopically, the cells all remained substantially intact after autoclaving, with approximate dimensions of 0.5 to 1 x 1 to 5 microns.

The killed bacterial slurry was diluted in water and administered by gastric lavage, daily for 7 days, to 5 mice, at a daily dose rate equivalent to 400 micrograms of bacterial dry matter.

A similar group of 5 mice serving as a control group received daily oral doses of physiological saline.

Both groups were then antigenically challenged with keyhole limpet haemocyanin with cholera toxin. Both groups were then assayed for immune globulins.

The IgA response in the group receiving *Bacillus subtilis* preparation exceeded that in the control group by a factor of 9.

In test B, the above procedure was repeated, except that the *Bacillus subtilis* preparation was allowed to stand at room temperature for a period of 72 hours, so that autolytic changes progressed to a stage of microscopically visible breakdown of the bacterial cells.

After the autoclaving, microscopy revealed that the bacterial preparation had broken down to a fine silt, of maximum particle size of 0.2 microns.

The antigenic challenge and subsequent assay of immune globulins was then carried out as in test A.

In test B, IgA response to the bacterial preparation was found to exceed that in the control group by a much reduced factor of 2.

Example 1 thus clearly demonstrates the beneficial effect of a killed bacterial preparation given orally as particles of 0.5 to 5 microns, compared to a similarly administered preparation of sub 0.2 micron particles.

### Example 2 (outside the scope of the present invention)

A comparison is made between two separate tests. In test C, chickens of from 1 to 21 days of age received food supplemented with a 2:1 methanol/chloroform extract of *Bacillus subtilis,* at the rate of the extract obtained from 100mg of *Bacillus subtilis* dried biomass per kg of feed.

The methanol/chloroform extract, substantially lipid in nature, was applied to a dusty and finely granular preparation of expanded mica containing a high proportion of particles of approximately 0.2 to 100 microns, before being incorporated into the feed. Growth rate of treated chickens exceeded that of controls by 14.1 per cent.

In test D, mice received a daily dose of a methanol/chloroform lipid extract of *Bacillus subtilis* administered by gastric lavage. The daily dose was the extract obtained from approximately 400mg of dried biomass. Compared to controls, no weight gain enhancement, or enhancement of immune response, was detected.

Example 2 clearly demonstrates the beneficial effect of using an orally administered bacterial preparation or extract in particulate form as compared to a non-particulate form.

### Example 3 (outside the scope of the present invention)

*Bacillus subtilis* cells, cultured and subjected to partial autolysis, as in Example 1, test A, were agitated for 48 hours in a mixture of 2 : 1 chloroform and methanol. Following centrifugation, the supernatant was concentrated by evaporation to yield a lipid extract.

The extract was combined with a liposome in a buffered saline solution to give dispersed particles of lipid/liposome of approximately 1 to 3 microns in diameter. This was administered to 5 mice by daily gastric lavage for 5 days, in a total daily dose per mouse of 0.2 ml, giving a total dose per mouse of 0.59 mg of extracted lipid. Five control mice received the same liposome preparation, but without the bacterial lipid extract, suspended in identical buffered saline and also administered at 0.2 ml per mouse per day.

On days 7 and 15, all the mice were immunised by gastric lavage with keyhole limpet haemocyanin (5 mg) in 200 mcl of physiological saline containing cholera toxin (10 µg). On day 21, all the mice were bled and mesenteric lymph nodes removed.

Tests by ELISA of specific antibodies in serum showed levels of IgA in the lipid extract treated mice to exceed the control mice by a factor of 8.

Cell cultures of mesenteric lymph nodes showed T cell proliferation in nodes of treated animals in response to keyhole haemocyanin to exceed that in control animals by factors of 35 and 16 following 3 and 5 days of culture.

The results therefore showed marked modulation of immune response through oral pre-treatment with bacterial lipid extracts given in particles of approximately 1 to 3 microns.

It should be noted that the method of extraction employed in Example 3 was specifically selected to extract only lipids from the bacteria. Lipopolysaccharides, such as those known as Lipid A, are not extractable by the method employed.

### Example 4 (outside the scope of the present invention)

Examples of a product in accordance with the invention comprise mixtures of approximately the same amounts of all the lipids comprising the aforesaid Group 1, and also of the aforesaid Group 2. All these lipids can be synthetically produced.

A further example tested was an extract of chloroform and methanol, (2:1 C/M) obtained from Bacillus subtilis cells, contained in liposomes formed according to the method of Bangham and Home (1964).

The same C/M extract of B. subtilis was also tested by oral administration of the extract applied to 1.5 micron particles of silicon dioxide. Lipids of Groups 1 and 2 were also administered orally in this way.

### Test Procedure

The aforesaid exemplary products have been tested on four groups of mice, orally treated with the products, and compared with two control groups. In all groups, measurements were made of systemic and mucosal immune responses to protein antigen (keyhole limpet hemocyanin -KLH) following treatment with the exemplary products.

### Materials and Methods

### Mice

Female Balb/c mice, 6-8 weeks old and weighing 20-22 gms were sued and maintained on a normal mouse diet throughout the study.

### Sample preparation

Samples were prepared for group treatments, as follows:
A) Physiological buffered saline (PBS) (Control treatment).
B) C/M extract of Bacillus subtilis, prepared in liposomes according to the method of Bangham and Home (1964).
C) C/M extract of Bacillus subtilis, applied to 1.5 micron particles of silicon dioxide.
D) Non-bacterial phospholipids (Group 1 above) applied to silicon dioxide particles as in C).
E) Non-bacterial neutral lipids and glycolipids (Group 2 above) applied to silicon dioxide particles as in C).
F) Liposomes prepared according to the method of Bangham and Horne (1964) with no contained test lipid or bacterial extract. (additional control treatment).

Preparations B, C, D and E provided daily oral doses per mouse of 53 micrograms of total lipids or chloroform/methanol lipid bacterial extract, made up with PBS to a total daily intra-gastric dose per mouse of 0.2 ml.

In the case of preparations C, D and E, lipids or bacterial extract were applied to 1.5 micron diameter approximately spherical particles of silicon dioxide according to the following method:-

### Preparation method for SiO₂ coating

The suspension matrix was prepared for the silicon dioxide beads via the following steps.
1. Sodium carbonate buffer was prepared
   100ml of 0.05 M NaHCO₃ solution was taken.
   The solution pH was adjusted to pH 9.7-9.9 with 0.1 M Na OH solution.
   The adjusted buffer was diluted to 200ml with sterilised water.
2. The suspension matrix was prepared.
   50ml of the Sodium carbonate buffer was taken and to this was added 100ml of Glycerine
   The two were mixed completely
3. The suspension matrix was then autoclaved.
   The suspension matrix was then complete.
   The lipids or extract were prepared for mixing with the silica via the following steps.
   All SiO₂ used in this method was 1.5 micron SiO₂ powder.
4. The extract sample was dissolved and mixed with SiO₂.
   The extract sample was weighed into a clean container.
   Every 25mg of extract was dissolved in 1ml of Chloroform/Methanol (2:1)
   The SiO₂ was weighed into the final container. 10mg of SiO₂ was used for every 5mg of extract.
   The extract solution was added to the SiO₂ and swirled to mix the two.
   The solvent was carefully removed by blowing with a stream of dry Nitrogen gas.
   The extract was now coating the SiO₂ and was ready for the suspension matrix.
5. The Phospholipid sample was dissolved and mixed with SiO₂.
   Every 25mg of lipid was dissolved in 1ml of Chloroform/Methanol (2:1)
   The SiO₂ was weighed into the final container. 10mg of SiO₂ was used for every 5mg of extract.
   The lipid solution was added to the SiO₂ and swirled to mix the two.
   The solvent was carefully removed by blowing with a stream of dry Nitrogen gas.
   The lipid was now coating the SiO₂ and was ready for the suspension matrix.
6. The Glycolipid sample was dissolved and mixed with SiO₂.
   Every 25mg of lipid was dissolved in 1ml of Chloroform/Methanol (2:1)
   The SiO₂ was weighed into the final container. 10mg of SiO₂ was used for every 5mg of extract.
   The lipid solution was added to the SiO₂ and swirled to mix the two.
   The solvent was carefully removed by blowing with a stream of dry Nitrogen gas.
   The lipid was now coating the SiO₂ and was ready for the suspension matrix.
7. The Neutral lipid sample was dissolved and mixed with SiO₂
   Every 25mg of lipid was dissolved in 1ml of Chloroform/Methanol (2:1)
   The SiO₂ was weighed into the final container. 10mg of SiO₂ was used for every 5mg of extract.
   The lipid solution was added to the SiO₂ and swirled to mix the two.
   The solvent was carefully removed by blowing with a stream of dry Nitrogen gas.
   The lipid was now coating the SiO₂ and was ready for the suspension matrix.
   The coated SiO₂ was suspended by the following steps
8. The SiO₂ was suspended by the matrix.
   For every 10mg of SiO₂ (and therefore 5mg of lipid) 10ml of the suspending matrix was used.
   The suspending matrix was added to the coated SiO₂.
   The mixture was shaken to loosen the coated SiO₂ from the walls of the vessel.
   The mixture in its sealed container was placed into an ultrasonic bath. The mixture was sonicated for 15 mins. This produced a fine dispersion of the coated SiO₂ within the matrix. The SiO₂ sometimes precipitated to the bottom of the container during this process or over time. Shaking the container re-suspended the SiO₂ into the matrix. If further sonication was required for complete dispersion, sonication was repeated for another 15 mins. It is necessary not to sonicate above the Tₘ (transition temperature) of the lipid. Doing so may result in large multilaminar vesicles rather than coating.

The suspension was now complete and ready for use.

### Immunisation schedule

There were 6 experimental groups, containing 5 mice each.
200µl of each preparation (or PBS or Liposome controls) was administered intragastrically to 6 groups of 5 mice, on 5 consecutive days.
On day 7 and day 17, mice from each group were immunised intragastrically with KLH (5mg) and cholera toxin (10µg) in 200µl PBS.

### Samples

Saliva samples were collected on day 20, following an intraperitoneal injection of 50µl pilocarpine (0.5 % w/v). On day 21 mice were killed and mesenteric lymph nodes removed.

### Preparation of single cell-suspension from mesenteric lymph nodes.

The mice were dissected and mesenteric lymph nodes (MLN) removed and placed in tissue culture medium, RPMI 1640. The MLN from each of the groups were pooled, homogenised and single cell suspension was obtained by passing the solution through a 70µm nylon mesh strainer.

The cells were then washed three times with RPMI containing 5% foetal calf serum at 4° C, 1200rpm. The cells were finally suspended in 1ml of complete media, counted in a haemocytometer with trypan blue to estimate viability and used for proliferation assays.

### T-cell proliferation assay

The cells were placed 8x10⁶/ml in triplicate in a 96 well tissue culture plate, with or without KLH at two concentrations 100µg/ml and 50µg/ml. The cells were incubated for 5 days at 37° C and were pulsed with 2µCi per well of titrated thymidine for the final 15 hours and then harvested onto filters and read in the 96 well matrix scintillation counter. Results were expressed as stimulation index i.e. the activity in antigen or mitogen stimulated wells divided by the activity in control (unstimulated) wells.

### ELISA assay

The specific anti-KLH IgA antibody content of each individual saliva sample was determined in an established ELISA assay and standardised against a positive control antiserum obtained by hyperimmunisation of mice with KLH (100µg) with cholera toxin (10µg) in Freund's incomplete adjuvant. Plates were coated with KLH at 2µg/ml and incubated with four twofold dilutions of samples (100µl) at 37°C for 2 hours. The initial dilution of saliva and vaginal samples was 1:10. Sheep anti-mouse IgA was used at 1:1000, for 2 hours at 37°C. Donkey anti-sheep IgG alkaline phosphatase conjugate was used at 1:250 for IgA assays for 1 hour at 37°C. The results were expressed as antibody units calculated from the standard curve obtained from the pooled immune mouse serum given an arbitrary antibody value of 100,000 IgA units/ml. The standard curve was diluted from 1:100 to 1:3200 for IgA samples. The value for each sample dilution falling on standard curve was calculated and the mean taken as the value for the sample.

### Results

### Cellular assay

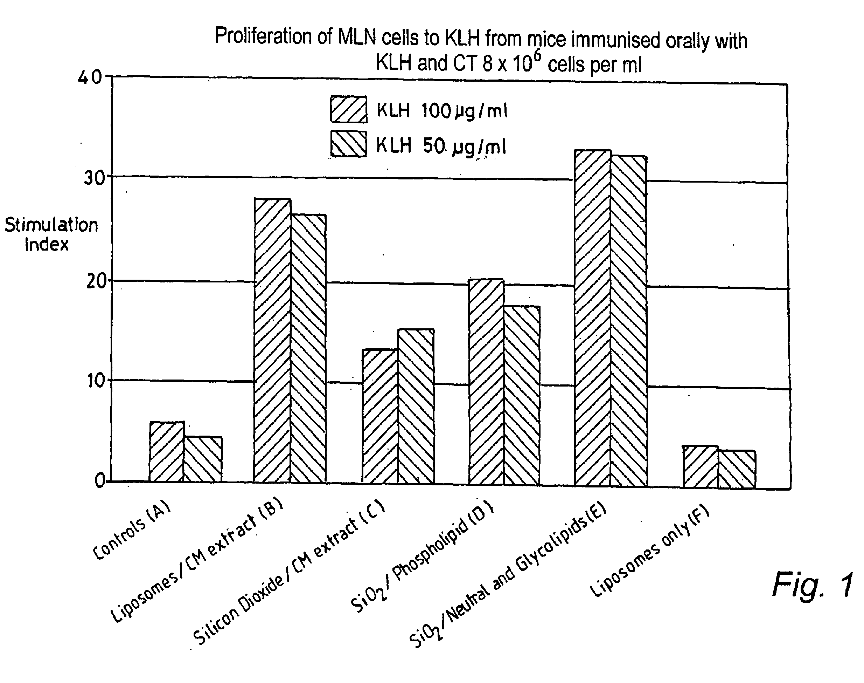

Mesenteric lymph node cells from the 5 mice within the group were pooled. The proliferation of MLN cells to KLH is significant in all the groups compared with controls (Fig 1). The stimulation index is enhanced in all experimental groups B-E but was particularly prominent for groups B and E. KLH used at the concentration of 100µh/ml and 50µg/ml had similar results.

### Mucosal antibodies

### Saliva:

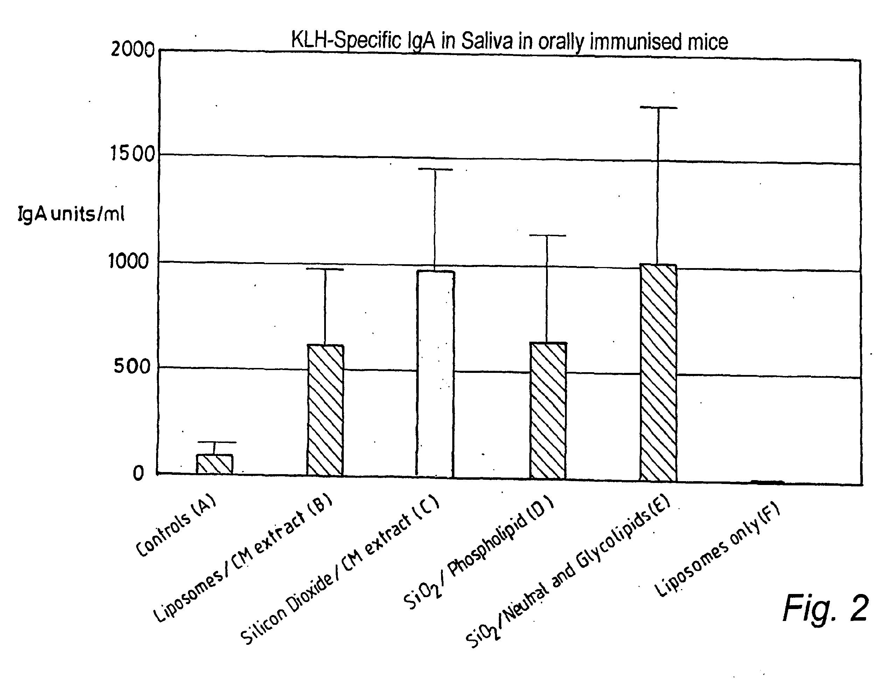

Oral immunisations with antigens resulted in salivary antibodies specific to KLH. Groups B, C, D and E show higher specific salivary IgA antibodies to KLH compared with controls (Fig 2).

### Conclusions from Example 4

1. Results confirm that the lipid bacterial extracts, given intra-gastrically in liposomes from 7 to 2 days before first immunisation, enhanced the cellular and antibody immune response.
2. The method of presenting the lipids (both extract and synthesised lipid) by coating them onto particles of silicon dioxide, before intra-gastric administration, has been shown to be effective in enhancing immune response to antigens.
3. Synthetic phospholipids (11 given simultaneously) and synthetic neutral and glycolipids (8 given simultaneously) have been shown to be effective in enhancing immune response to antigens, when given by the methods described.

### Example 5 (outside the scope of the present invention)

This is a comparison of treatments in Example 2 (Test A) and Example 3 (Test B).

Comparison is made between two tests in mice where MLN T-cell proliferation response to KLH was assayed by the method described in Example 4 (Cellular assay).

In both tests, Bacillus subtilis was grown in a clear liquid medium containing yeast extract, sucrose, magnesium and other soluble nutrients, according to well established procedures.

The cells were separated from the culture by centrifugation, subjected to partial autolysis and lipid was extracted by agitation for 48 hours in a mixture of 2;1 chloroform and methanol. Following centrifugation, the supernatant was concentrated by evaporation to yield lipid extract.

In Test A the lipid extract was dispersed in an aqueous medium using a commercially available emulsifying agent (Crillet 4) and administered to 5 mice by gastric lavage, daily for 5 consecutive days, at a dose rate per mouse per day of 53 micrograms of extract on 0.2 ml of PBS .

In Test B, lipid extract was prepared and administered to mice of the same age and strain (young female Balb C) by the same route and at the same dosage, but incorporated in liposomes of the bilosome type (method of J. Brewer, Strathclyde University) of approximately 1-3 microns diameter.

In both tests similar groups of control mice received daily intra-gastric administrations of 0.2ml PBS.

On days 7 and 25 mice from both treated and control groups in both tests were immunised intragastrically with KLH (5 micrograms) and cholera toxin (10 micrograms) as in Example 1.

Mesenteric lymph nodes were removed on day 21, and tests of T-cell proliferative response to KLH were performed as in Example 1.

Results were determined in terms of 3 H thymidine incorporation, and expressed in CPM above backgrounds.

In Test A, where the lipid extract was given in non-particulate form, cells from the treated mice showed no proliferative response after 3 days incubation, compared with 37,000 cpm above background for controls. In this case, the T-cell proliferative response was therefore strongly depressed by the treatment.

In Test b, where the lipid extract was given in particulate form in liposomes, the treated mice showed a proliferative response after 3 days incubation of 140,000 cpm above background, compared with 12,000 cpm for controls. In this case, the T-cell proliferative response was clearly strongly increased by the treatment.

This example shows that the effect of lipids applied to mucosal surfaces upon the animal's immune response to antigens is profoundly affected by the physical from in which the lipids are administered. Depending upon the physical from, the immune response to antigens may be increased or suppressed.

### Example 6

In a further test, two groups of mice as employed in Example 2, were immunised by two administrations of antigen, 14 days apart, by the intraperitoneal and intragastric routes. These administrations were accompanied by concurrent administrations of Group 2 lipids (glycolipids and neutral lipids) as employed in Example 4.

Materials employed use:

### Intraperitoneal administration

**Synthetic lipids as in Example 4,**

| | |
|---|---|
| Group 2 | 0.14 microgram in 50mcl aqueous dispersion |
| KLH | 0.5 micrograms in 50mcl aqueous dispersion |

### Intragastric administration

Synthetic lipids as in Example 4,

| | |
|---|---|
| Group 2 | 14 micrograms on SiO₂ particles in 0.2ml PBS |
| KLH | 5 milligrams in 0.2ml PBS |

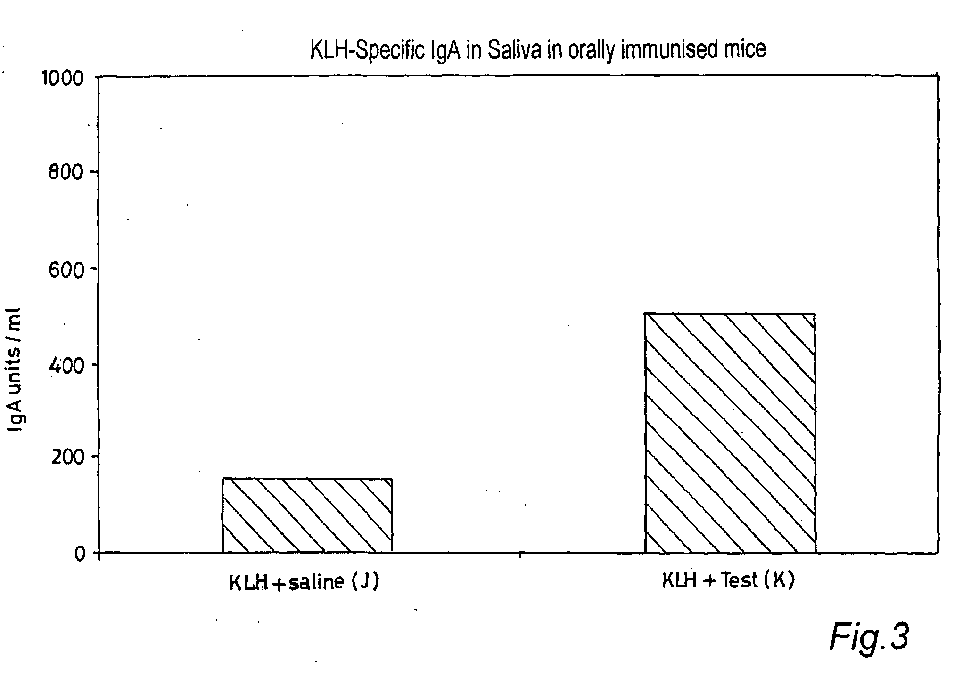

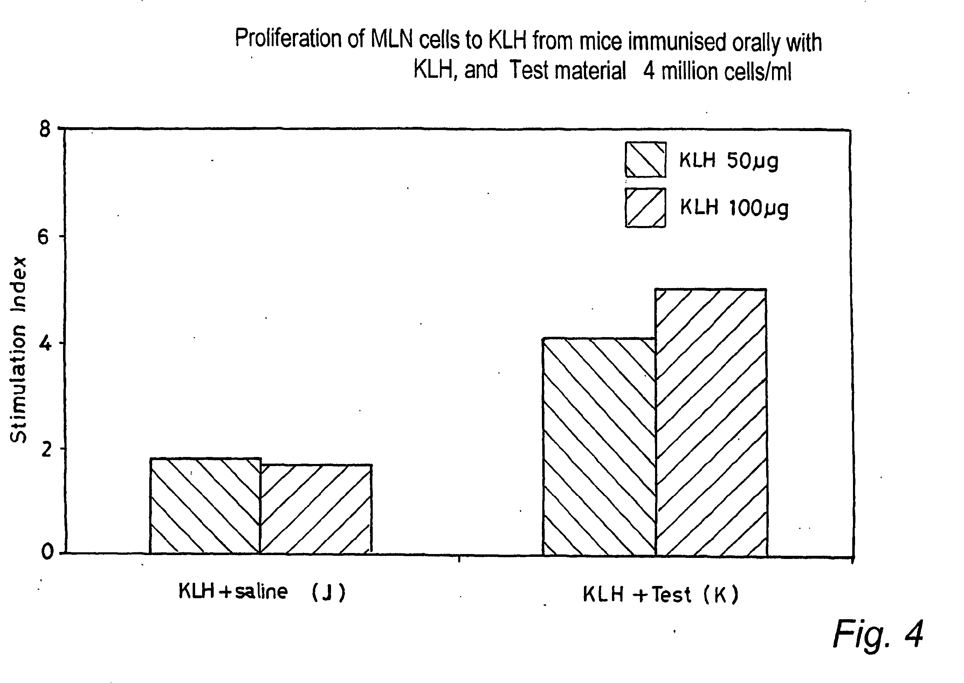

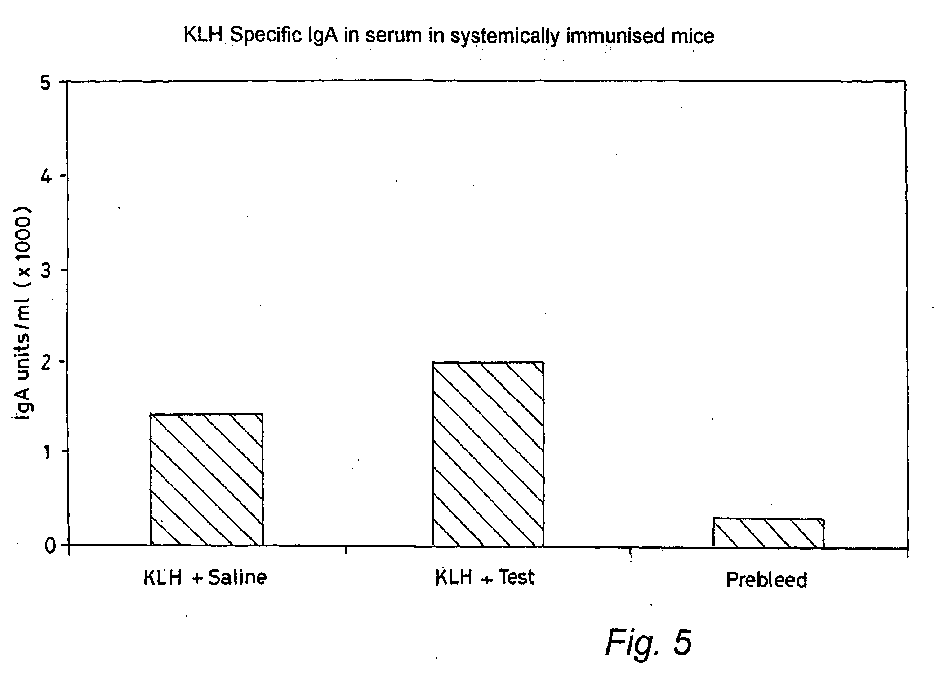

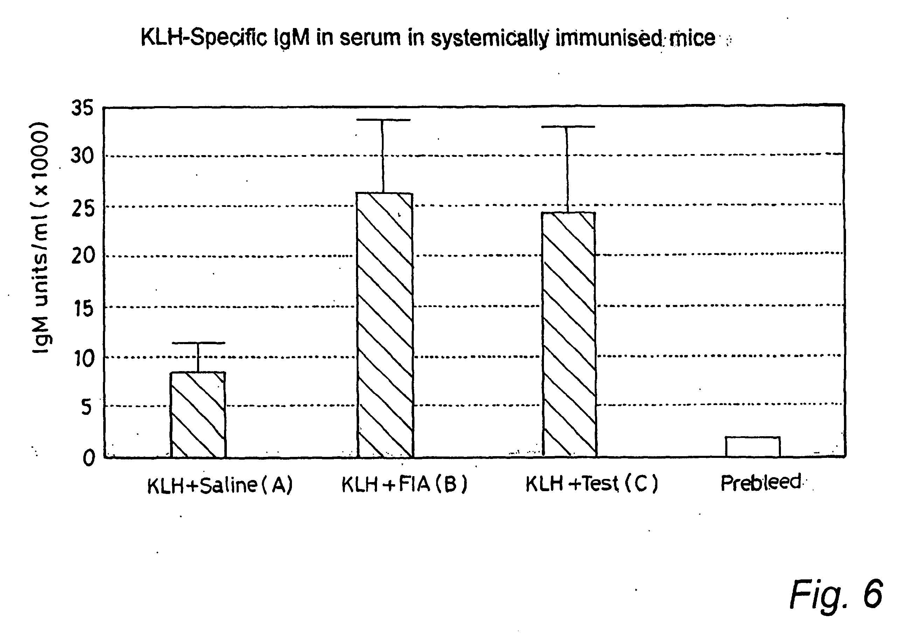

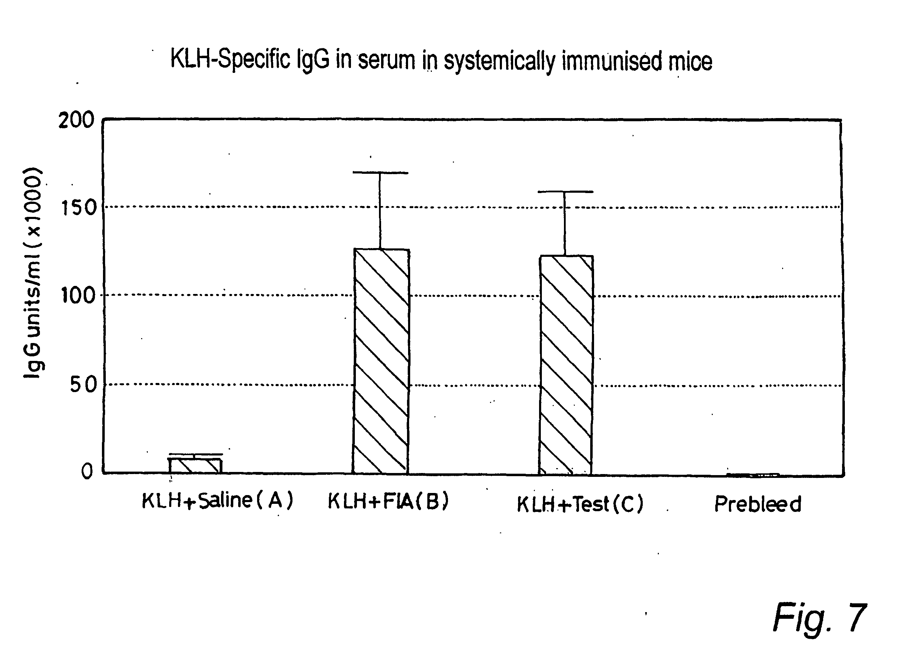

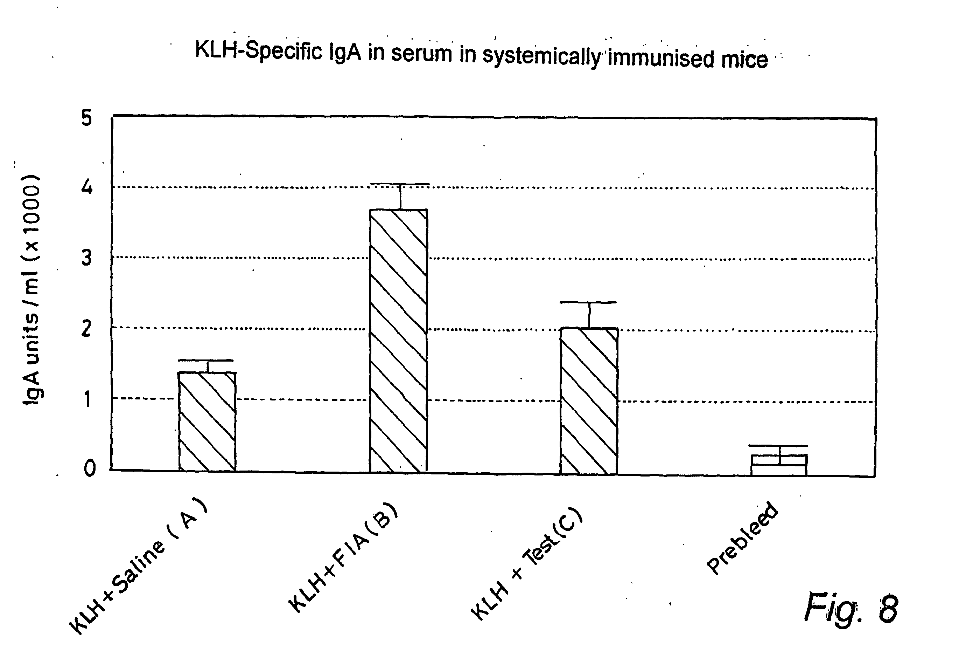

### Results of Example 6 are given in Figures 3, 4 and 5

The results show that Group 2 lipids can be employed as adjuvants, given concurrently with antigens, both parenterally and via the gastro-enteric mucosa.

### Example 7 (outside the scope of the present invention)

The objective of this study was to investigate the adjuvanticity of 19 different lipids.

### Materials and Methods

### Mice

Female Balb/c mice, 6-8 weeks and weighing about 20-22gms were used and maintained on a normal mouse diet throughout the study.

### Lipid preparations

The following lipid preparations were prepared:-

**List of abbreviations:**

| | | |
|---|---|---|
| C | PAM | (L-*a*-Phosphatidicacid, dimyristoyl) |
| D | PAH | (L-*a*-Phosphatidicacid, diheptadecanoyl) |
| E | PCM | (DL-*a*-Phosphatidylcholine, dimyristoyl) |
| F | PCP | (L-*a*-Phosphatidylcholine, dipentadecanoyl) |
| G | PCH | (L-*a*-Phosphatidylcholine, diheptadecanoyl) |
| H | PCS | (DL-*a*-Phosphatidylcholine, distearoyl) |
| I | PEM | (L-*a*-Phosphatidylethanolamine, dimyristoyl) |
| J | PEP | (DL-*a*-Phosphatidylethanolamine, dipalmitoyl) |
| K | PES | (L-*a*-Phosphatidylethanolamine, distearoyl) |
| L | PEH | (L-*a*-Phosphatidylethanolamine, diheptadecanoyl) |
| M | PSP | (DL-*a*-Phosphatidyl-L-serine, dipalmitoyl) |
| N | PS | (N-Palmitoyl-*d*-sphigosine) |
| O | PC | (N-Palmitoyl dihydroglucocerebroside) |
| P | TM | (Trymyristin) |
| Q | Tpen | (Tripentadecanoin) |
| R | Tpal | (Tripalmitin) |
| S | DPM | (1,2 Diplamitoyl-3-myristoyl-rac-glycerol) |
| T | DSM | (1,2 Distearloyl-3-myristoyl-rac-glycerol) |
| U | DSP | (1,2 Distearoyl-3-palmitoyl-rac-glycerol) |
| V | MIX | (Cocktail of above lipids) |

### Experimental Groups and immunisation schedule

There were 22 groups of mice marked alphabetically from A-V. Group A (8 mice) was the negative control (KLH+Saline), Group B (8 mice) was the positive control (KLH+IFA). Groups C-V were the experimental groups containing 6 mice each. 100µl of antigen preparation comprising of 5µl lipid + 45µl saline + 50µl (2mg/ml KLH) was administered to 20 groups of 6 mice each, on week 0 followed by a boost on week 2.

### Samples

On day 7 and day 21 after the booster dose, the mice were tail bled, the serum separated, aliquoted and stored at 4°C

### ELISA assay

The specific anti-KLH IgG and IgM antibody content of each individual serum sample was determined in an established ELISA assay and standardised against a positive control antiserum obtained by hyperimmunisation of mice with KLH (100µg) in Freund's incomplete adjuvant. Plates were coated with KLH at 2µg/ml and incubated with four twofold dilutions of samples (100µl) at 37°C for 2 hours. The initial dilution of serum samples was 1:50 for IgA assay and 1:100 for IgG and IgM assays. Sheep and mouse IgA and IgM were used at 1:1000 and IgG at 1:2000 for 2 hours at 37°C. The results were expressed as antibody units calculated from the standard curve obtained from pooled immune mouse serum given an arbitrary antibody value of 100,000 IgA units/ml and 1 x 10⁶ IgG and IgM units/ml. The standard curve was diluted from 1:100 to 1:3200 for IgA, 1:4000 to 1:256,000 for IgG and 1:1000 to 1:32000 for IgM samples. The value for each sample dilution falling on standard curve was calculated and the mean taken as the value for the sample.

### Results

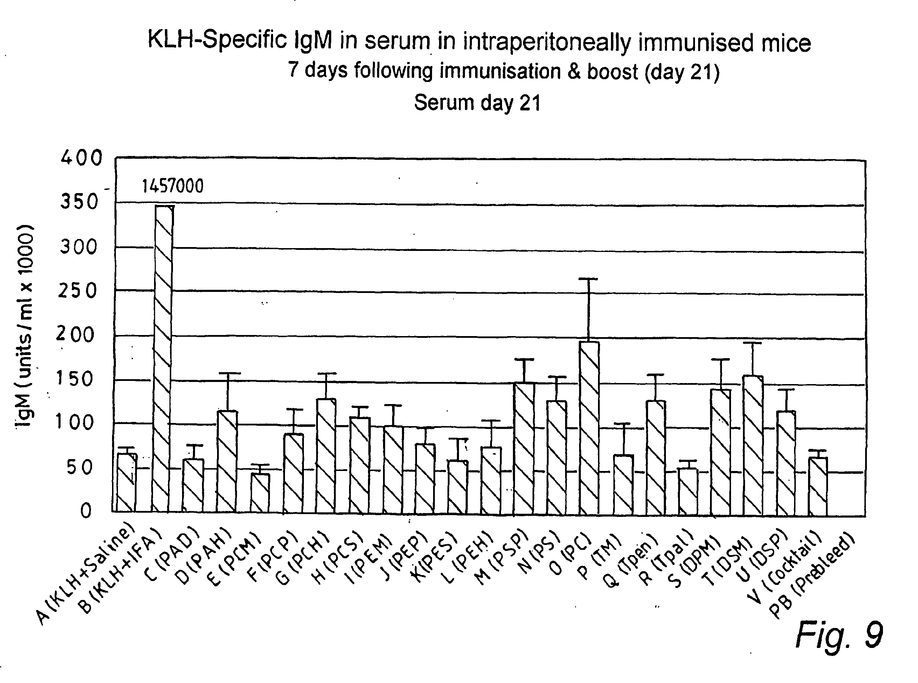

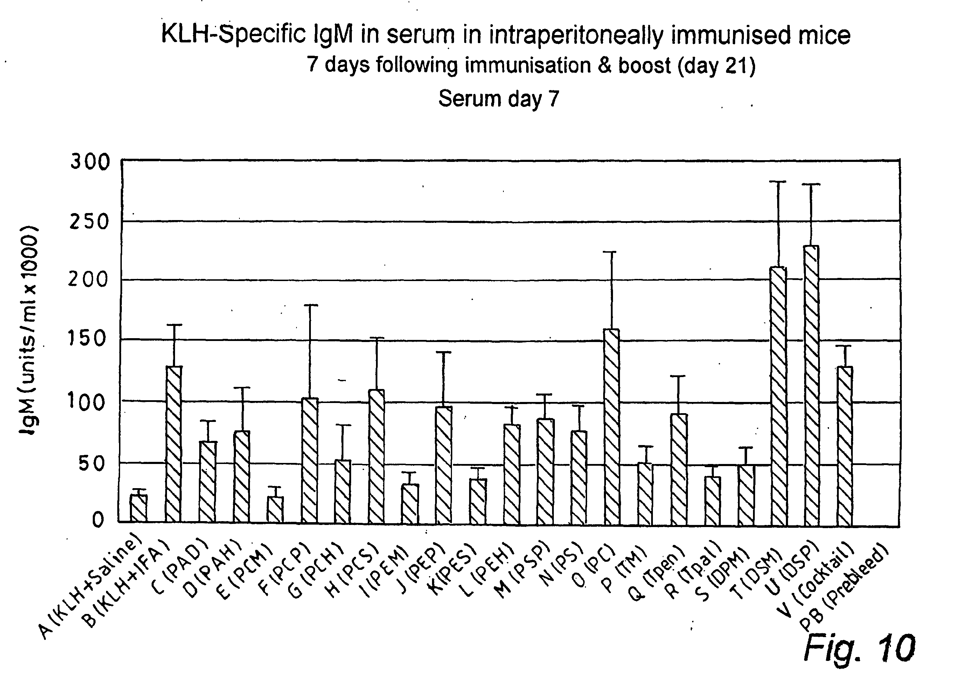

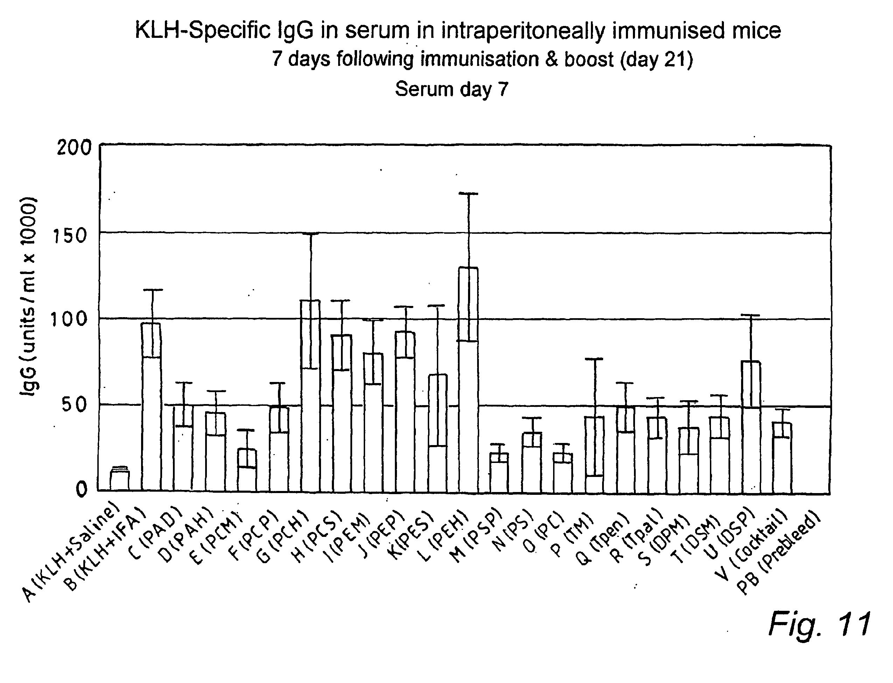

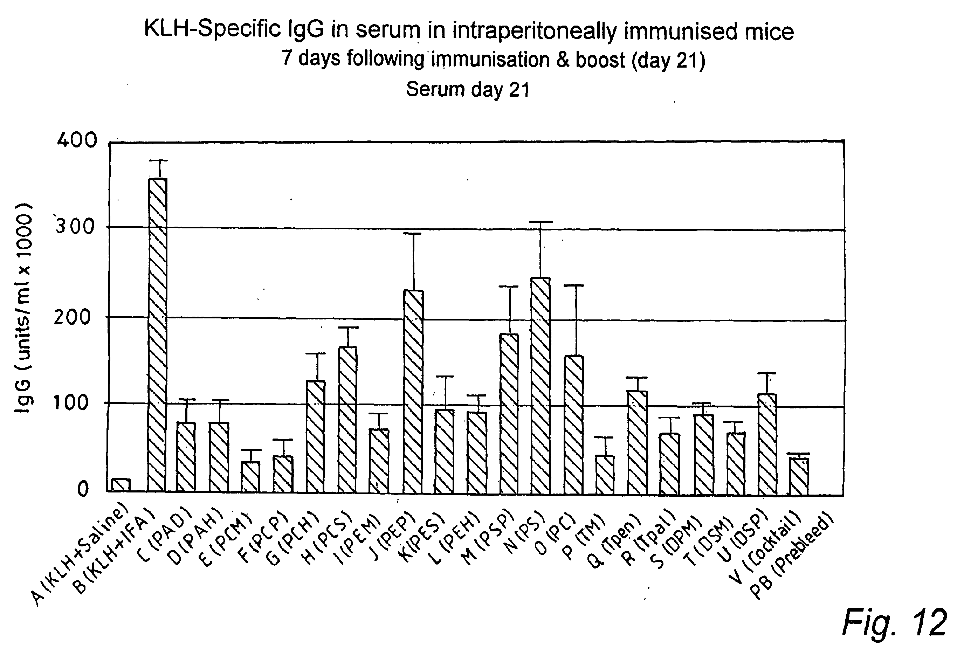

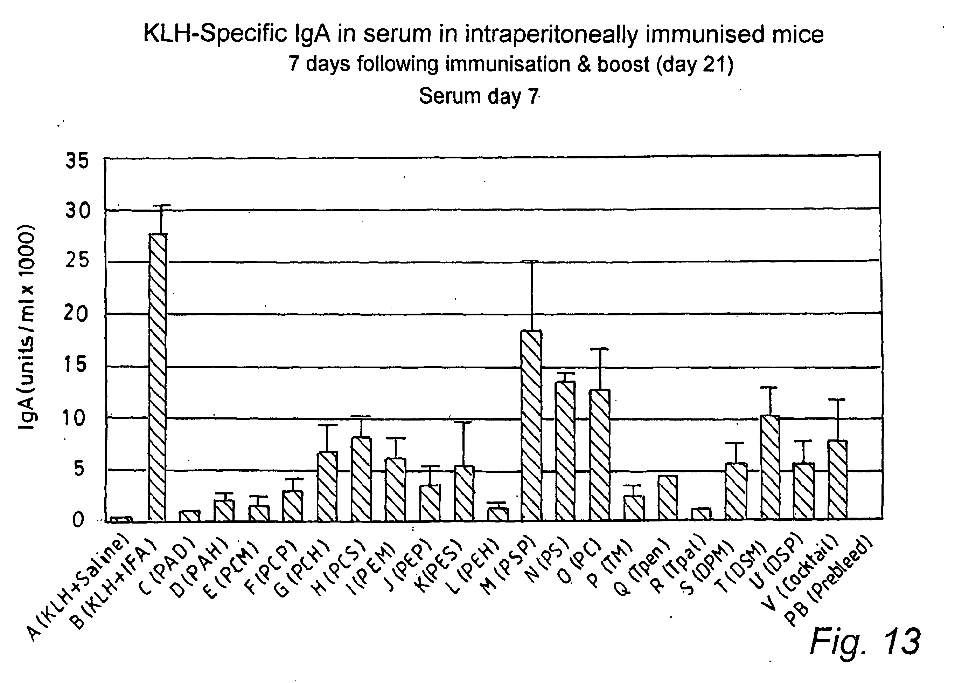

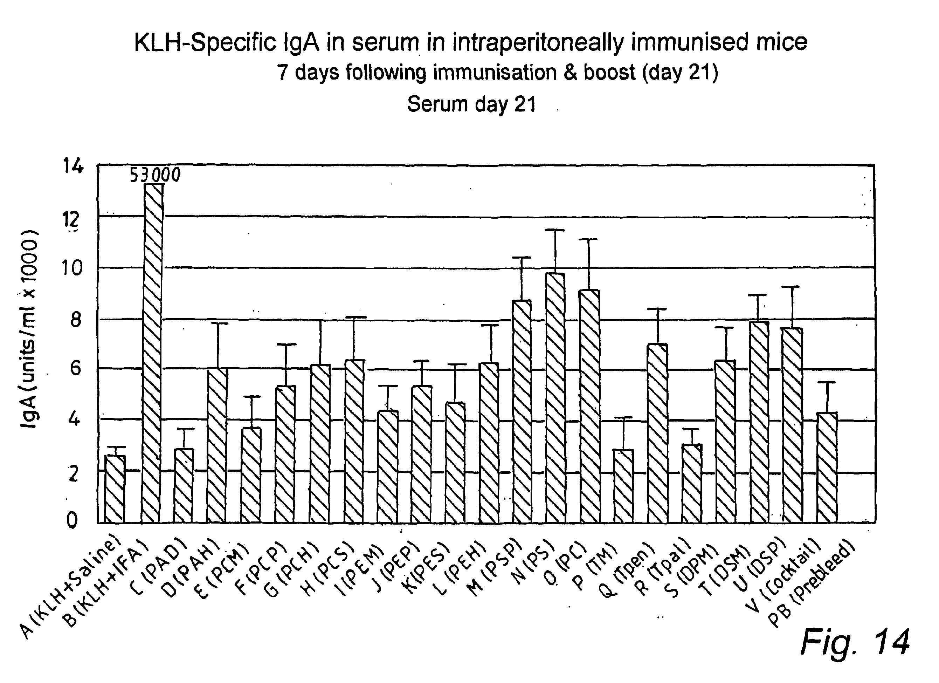

Results are given in the form of histograms (Figs 9 to 14), plotting the Ig response (units/ml) against the trial grouping. From these data each trial group was given a numerical score based upon its performance compared to the control. The number signifies multiples of the control response.

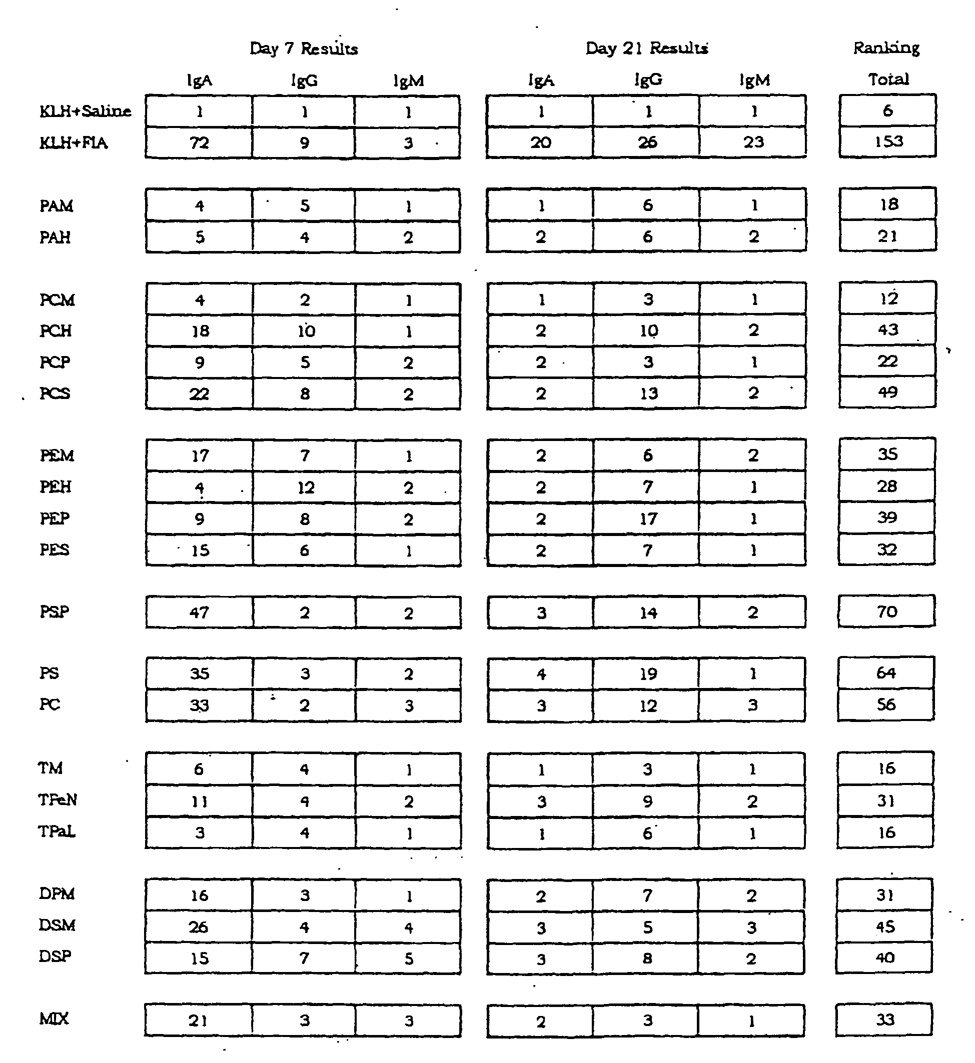

### Results

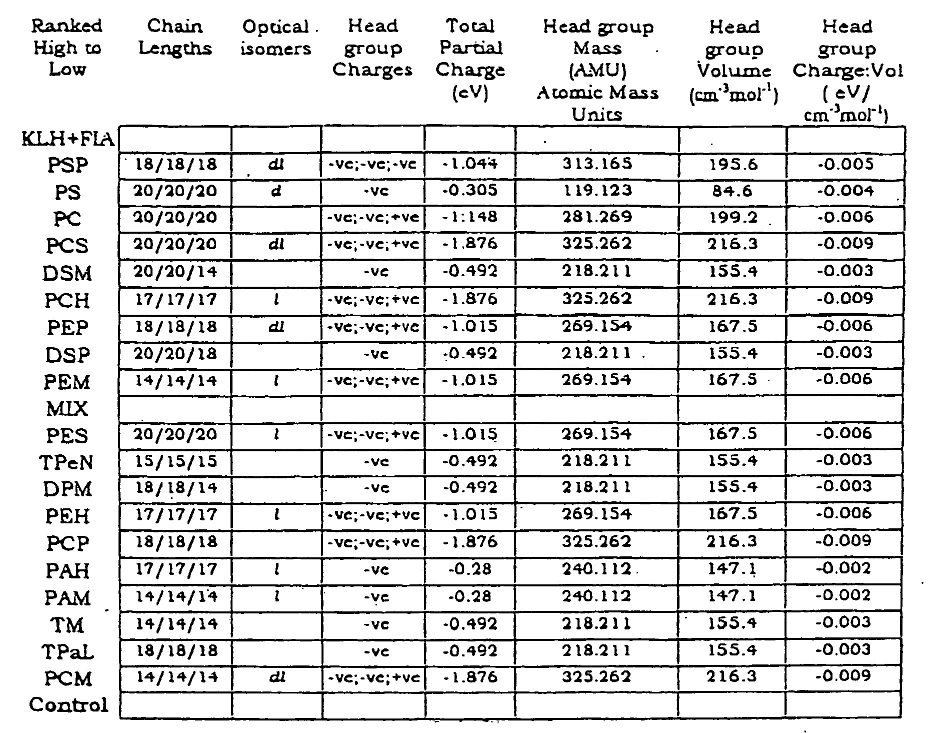

### Observations

The trends within the test are as follows:

### Chain Uniformity

When multiple chains are present on a lipid it is preferable to have at least two different lengths on the same lipid molecule.

This is shown by comparison of DSP, DSM against TpaL and TM. Both DSP and DSM have mixed chain lengths, and the resulting performance is good (Ranking of 39 and 45 respectively). TpaL and TM each have three fatty acid chains that are the same length. These are comparable in length to those on DSP and DSM. Their performance in the trial was poor (Ranking of 15 and 17 respectively).

### Optical isomerism

When a lipid shows optical isomerism it is preferable to use the d isomer.

This can be shown by the comparison of PEP against PEH. Both are structurally similar, however PEP, which contains a d isomer, performs better than PEH that only contains an 1 isomer (Ranking 40 and 28 respectively).

### Chain length

The length of the fatty acid chain is important as a governing principle. It is preferable to have a chain length that consists of more than 14 carbon atoms and ideally 18 to 20 carbon atoms.

This can be shown by the comparison of PEP and PSP against PAM and PCM. Both PEP and PSP have fatty acid chains containing 18 carbon atoms and both perform well (Ranking 40 and 70 respectively); PAM and PCM both have chains that contain 14 carbon atoms and perform poorly in the experiment (Ranking 18 and 12 respectively).

### Head group structure

The size of the head group is important. It is preferable to have a large (both in volume and mass) head group. Ideally the mass should be 269 AMU or above and the volume should be 167 cm⁻³mol⁻¹ or above.

This can be shown by the comparison of PEP and PSP against PAM and TM. Both PEP and PSP have large head groups. Both these lipids perform well (Ranking 40 and 70 respectively). Both PAM and TM have smaller head groups; both of these lipids perform poorly (Ranking 18 and 17 respectively).

The charge of the head group is important. It is preferable for the head group to have a combined partial charge of -1.015 eV or more strongly negative. Ideally the combined partial charge should be -1.044 eV or more strongly negative.

This can be shown by the comparison of PEP and PSP against PAM and TM. Both PEP and PSP have strongly negative head groups; both these lipids perform well (Ranking 40 and 70 respectively). Both PAM and TM have weakly charged head groups; both of these lipids perform poorly (Ranking 18 and 17 respectively).

Where the head group has a partial charge it is preferable for the charge to be in multiple centres.

This can be shown by the comparison of PEP and PSP against PAM and TM. Both PEP and PSP have head groups with multiple charge centres. Both these lipids perform well (Ranking 40 and 70 respectively). Both PAM and TM have head groups with a single charge centre; both of these lipids perform poorly (Ranking 18 and 17 respectively).

The head group charge to volume ratio should be relatively large. It is preferable to have a charge of -0.004 eV/cm⁻³mol⁻¹ or more per unit. Ideally the charge to volume ration should be -0.006 eV/ cm⁻³mol⁻¹ or more strongly negative.

This can be shown by the comparison of PEP and PSP against PAM and TM. Both PEP and PSP have head groups with stronger negative charge to volume ratios. Both these lipids perform well (Ranking 40 and 70 respectively). Both PAM and TM have head groups with weak negative charge to volume ratios; both of these lipids perform poorly (Ranking 18 and 17 respectively).

### Example 8

The objects of this experiment were:
a) To test the effects of unsaturated lipids compared to closely similar saturated analogues on enhancing immune response to antigens.
b) To test the effect of naturally occurring lipids of plant origin, administered orally in particulate form, on enhancing said response.

### Materials and Methods

7 groups of 6 mice as employed in Example 4, were immunised by three administrations of test lipids on three consecutive days (Days 1, 2 and 3). On Dyas 7 and 14 mice were immunised intragastrically with KLH (5mg) in 200µl saline containing cholera toxin (10) as employed in Example 4.

### Materials employed:-

**Intragrastric lipid administration**

| | |
|---|---|
| Synthetic lipids as in Example 4 (See Fig. 15, PHOS.) | 40 µg on SiiO₂ particles in 0.2ml PBS, per mouse per day |
| Phosphatidylcholine, distearoyl (PCS) | 40 µg on SiO₂ particles in 0.2ml PBS, per mouse per day |
| Phosphatidylcholine, dioleoyl (PCO) | 40 µg on SIO₂ particles in 0.2ml PBS, per mouse per day |
| Trimyristin^{™} | 40 µg on SiO₂ particles in 0.2ml PBS, per mouse per day |
| Trilinolenin (TL) | 40 µg on SiO₂ particles in 0.2ml PBS, per mouse per day |
| Coconut, Palm and Cottonseed Oil (COM) | 40 µg on SiO₂ particles in 0.2ml PBS, per house per day |

The mice were killed on Day 21. Samples of mesenteric lymph nodes were taken for assays of proliferative cellular response to KLH.

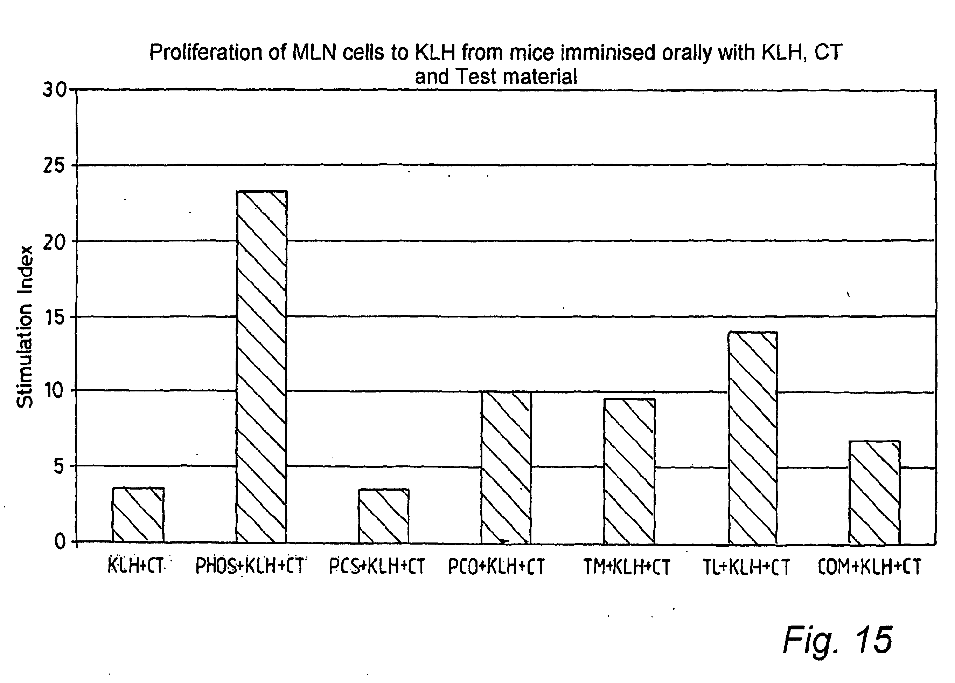

Results are given in Figure 15.

Comparison of the control treatment receiving no lipid pre-treatment KLH+CT with the positive control receiving phospholipid pre-treatment PHOS+KHL+CT) previously shown to be effective in Example 4) confirms validity of this test. The results show camparison between Phosphatidycholine, distearloyl, a saturated lipid that showed good performance in Example 7, and Phosphatidycholine, dioleoyl, as structurally similar unsaturated analogue. Also compared within these results are Trimyristin, a saturated lipid that showed poor performance in Example 7, and Trilinolenin, a structurally similar unsaturated analogue. Both of these comparisons show that the unsaturated analogues performed better than their unsaturated equivalents giving a strong proliferative response compared to control. These results therefore confirm the effectiveness of unsaturated lipids in enhancing the immune response. Also, this example shows that lipids of plant origin are useful in achieving this purpose.

Trial group COM, receiving plant based lipids selected to give comparable content to synthetic lipids as employed in Example 4, Group 1, showed a strong proliferative response when compared to the control group. In this case the T-cell proliferative response was strongly increased by the treatment.

## Claims

1. Use of a synthetic lipid or lipid from animal or vegetable oil, said lipid having a glycerol backbone carrying at least one alkyl or acyl chains, wherein the lipid is phospholipid, glycolipid or neutral lipid, and the number of carbon atoms in the hydrocarbon chains is between 10 and 22 inclusive, in the preparation of a composition to stimulate and/or modulate in a human or animal subject an immune response to an antigen; wherein the composition is adapted to be given to the subject in non-particulate or particulate form, by parenteral or transmucosal administration, and wherein the lipid/s present in the composition is **characterised by** the following:-
a) one or more fatty acid chains, each having a chain length of 10 to 22 carbon atoms, preferably 12 to 20 and more preferably 16 to 20 or even 18 to 20;
b) where the lipid has a head group, the head group has a mass of at least 269 Atomic Mass Units (AMU) and a volume of at least 167 cm⁻³mol⁻¹, and a combined partial electrical charge of -1.015eV or more strongly negative, and preferably of -1.044eV or more strongly negative, and wherein the head group may have a single charge centre, but preferably has multiple charge centres; and
c) where the lipid has a head group, the head group charge to volume ratio is preferably -0.004.eV/cm⁻³mol⁻¹ or more strongly negative, and more preferably -0.006eV/cm⁻³mol⁻¹ or more strongly negative.

2. A use according to claim 1, wherein the composition is adapted to be administered transmucosally and comprises lipid in particulate form.

3. A use according to claims 1 or 2, wherein the composition comprises lipid rendered into a particulate form by incorporation into or onto liposomes or bilosomes or other appropriately sized particulate matter, such as silica, titanium dioxide, or kaolin particles.

4. A use according to claim 3, wherein the lipid or lipids are rendered into a liquid of low viscosity, as by heating, dissolving in solvents such as chloroform or methanol, or by forming into an aqueous preparation by means of an emulsifying agent, to form a emulsion or colloid, and particles to be coated are then dispersed in the liquid by stirring, shaking vibration, sonication or other known means of dispersion, the liquid then being removed to leave a coating of the lipid or lipids on the particle surfaces.

5. A use according to claim 4, wherein the liquid is removed by evaporation of solvents or aqueous medium, centrifugation (batch or continuous flow) or filtration, preferably cross-flow filtration.

6. A use as claimed in claim 3 or claim 4, wherein the lipid is shown, for instance by microscopic examination, to be applied to the particles, and a carrier may be added in suitable volume to provide a convenient dosage form.

7. A use according to any one of claims 3 to 5, wherein the lipid is incorporated into particulate formulations of wet or dry powder or granular mixes, or alternatively incorporated into liquids such as water, oil, milk or any beverage.

8. A use according to any one of claims 3 to 5, wherein the composition is adapted to be administered as a tablet, capsule or paste, or incorporated into foods.

9. A use according to claim 5, wherein the particles have a size range of 0.1 to 100 microns, preferably 0.1 to 25 microns and most preferably 0.2 to 15 or even 0.3 to 10 microns, and ideally 0.4 to 8 microns or 0.5 to 5 microns.

10. A use according to claim 1, wherein the composition is adapted to be administered parenterally or transmucosally in particulate form with the particles having dimensions 0.1 microns to 100 microns, more specifically 0.2 to 25 microns, preferably 0.2 microns up to 15 microns or even 0.3 up to only 10 microns and ideally 0.4 to 8 microns or 0.5 to 5 microns.

11. A use according to any one of claims 1 to 10, wherein the lipids are phospholipids, glycolipids or neutral lipids, saturated or unsaturated, with or without branching in their fatty acid chains, within the categories of:-
Phosphatidic acids
Phosphatidylethanolamines
Phosphatidylserines
Phosphatidylinositols
Phosphatidylcholines
Cardiolipins
Triglycerides
1,2-Diglycerides
1,3-Diglycerides and
Monoglycerides

12. A use according to claim 11, wherein the lipids include any one or more of the following:-
Phosphatidylcholine, dioleoyl
Trilinolenin
L-a-Phosphatidicacid,
Dimyristoyl L-*a*-Phosphatidicacid,
Diheptadecanoyl DL-*a*-Phosphatidylcholine,
Dimyristoyl L-*a*-Phosphatidylcholine,
Dipentadecanoyl L-*a*-Phosphatidylcholine,
Diheptadecanoyl DL-*a*-Physphatidylcholine,
Distearoyl L-*a*-Phosphatidylethanolamine,
Dimyristoyl DL-*a*-Phosphatidylethanolamine,
Dipalmitoyl 1,2-Distearoyl-sn-glycero-3-phosphoethanolamine
L-α-Phosphatidylethanolamine,
Diheptadecanoyl DL-*a*-Phosphatidyl-L-serine,
Dipalmitoyl N-Palmitoyl-d-sphingosine
N-Palmitoyl dihydroglucocerebroside
Trymyristin
Tripentadecanoin
Tripalmitin
1,2 Dipalmitoyl-3-myristoyl-rac-glycerol
1,2 Distearoyl-3-myristoyl-rac-glycerol
1,2 Distearoyl-3-palmitoyl-rac-glycerol and
rac-1,2-Dimyristoyl-3-palmitoylglycerol.

## Patentansprüche

1. Verwendung eines synthetischen Lipids oder eines Lipids aus einem tierischen oder pflanzlichen Öl, wobei das Lipid ein Glycerin-Rückgrat aufweist, das mindestens eine Alkyl- oder Acyl-Kette trägt, wobei das Lipid ein Phospholipid, Glycolipid oder ein neutrales Lipid ist, und wobei die Anzahl der Kohlenstoffatome in den Kohlenwasserstoffketten zwischen 10 und 22 einschließlich beträgt, zur Herstellung einer Zusammensetzung, um in einem menschlichen oder tierischen Probanden eine Immunantwort gegenüber einem Antigen zu stimulieren und/oder zu modulieren; wobei die Zusammensetzung so eingestellt ist, dass sie dem Probanden in einer nichtpartikulären oder partikulären Form durch parenterale oder transmucosale Verabreichung gegeben wird, und wobei das (die) in der Zusammensetzung vorliegenden Lipid(e) durch die folgenden Merkmale **gekennzeichnet** wird (werden):
a) eine oder mehrere Fettsäureketten, die jeweils eine Kettenlänge von 10 bis 22 Kohlenstoffatomen, vorzugsweise von 12 bis 20 und stärker bevorzugt von 16 bis 20 oder auch von 18 bis 20 aufweisen;
b) wobei das Lipid eine Kopfgruppe aufweist, wobei die Kopfgruppe eine Masse von mindestens 269 atomaren Masseneinheiten (AMU) und ein Volumen von mindestens 167 cm⁻³mol⁻¹, und eine kombinierte, elektrische Partialladung von -1,015 eV oder stärker negativ aufweist, und bevorzugt von -1,044 eV oder stärker negativ, und wobei die Kopfgruppe ein einzelnes Ladungszentrum aufweisen kann, jedoch bevorzugt vielfache Ladungszentren aufweist; und
c) wobei das Lipid eine Kopfgruppe aufweist, wobei das Verhältnis der Ladung zum Volumen der Kopfgruppe vorzugsweise -0,004 eV/cm⁻³mol⁻¹ oder stärker negativ, und stärker bevorzugt -0,006 eV/cm⁻³mol⁻² oder stärker negativ ist.

2. Verwendung nach Anspruch 1, wobei die Zusammensetzung so eingestellt ist, dass sie transmucosal zu verabreichen ist, und ein Lipid in partikulärer Form umfasst.

3. Verwendung nach Anspruch 1 oder 2, wobei die Zusammensetzung ein Lipid umfasst, das zu einer partikulären Form durch Einbau in oder auf Liposomen oder Bilosomen oder ein anderes partikuläres Material von geeigneter Größe, wie z.B. Siliziumdioxid, Titandioxid oder Kaolinpartikel, gemacht wurde.

4. Verwendung nach Anspruch 3, wobei das Lipid oder die Lipide zu einer Flüssigkeit von niedriger Viskosität gemacht werden, wie durch Erhitzen, Auflösen in Lösungsmitteln, wie z.B. Chloroform oder Methanol, oder indem es zu einer wässrigen Zubereitung mittels eines emulgierenden Mittels geformt wurde, um eine Emulsion oder ein Kolloid zu bilden, und wobei die zu beschichtenden Teilchen anschließend in einer Flüssigkeit durch Rühren, schüttelnde Schwingungen, Beschallung oder durch andere bekannte Mittel zur Dispergierung dispergiert werden, und die Flüssigkeit anschließend entfernt wird, um eine Beschichtung aus dem Lipid oder den Lipiden auf den Partikeloberflächen zurückzulassen.

5. Verwendung nach Anspruch 4, wobei die Flüssigkeit durch die Verdampfung von Lösungsmitteln oder eines wässrigen Mediums, durch Zentrifugation (im Chargenbetrieb oder im kontinuierlichen Betrieb) oder durch Filtration, vorzugsweise durch Querstrom-Filtration, entfernt wird.

6. Verwendung nach Anspruch 3 oder Anspruch 4, wobei gezeigt wird, z.B. durch mikroskopische Prüfung, dass das Lipid auf die Teilchen aufgetragen wurde, und wobei ein Träger in einem geeigneten Volumen zugegeben werden kann, um eine zweckmäßige Dosierungsform bereitzustellen.

7. Verwendung nach einem der Ansprüche 3 bis 5, wobei das Lipid in partikuläre Formulierungen eines feuchten oder trockenen Pulvers oder von körnigen Mischungen eingearbeitet wird, oder alternativ in Flüssigkeiten eingearbeitet wird, wie z.B. Wasser, Öl, Milch oder ein beliebiges Getränk.

8. Verwendung nach einem der Ansprüche 3 bis 5, wobei die Zusammensetzung so eingestellt wird, dass sie als eine Tablette, Kapsel oder Paste oder im Nahrungsmittel eingearbeitet zu verabreichen ist.

9. Verwendung nach Anspruch 5, wobei die Partikel einen Größenbereich von 0,1 bis 100 µm, vorzugsweise von 0,1 bis 25 µm, und am meisten bevorzugt von 0,2 bis 15, oder auch von 0,3 bis 10 µm, und idealerweise von 0,4 bis 8 µm oder von 0,5 bis 5 µm aufweisen.

10. Verwendung nach Anspruch 1, wobei die Zusammensetzung so eingestellt wird, dass sie parenteral oder transmucosal in partikulärer Form zu verabreichen ist, wobei die Partikel-Abmessungen von 0,1 µm bis 100 µm, insbesondere von 0,2 bis 25 µm, vorzugsweise von 0,2 µm bis 15 µm, oder auch von 0,3 bis lediglich 10 µm, und idealerweise von 0,4 bis 8 µm oder von 0,5 bis 5 µm aufweisen,

11. Verwendung nach einem der Ansprüche 1 bis 10, wobei die Lipide Phospholipide, Glycolipide oder neutrale Lipide, gesättigte oder ungesättigte Lipide, solche mit oder ohne Verzweigung in ihren Fettsäureketten sind, wobei sie aus folgenden Kategorien ausgewählt werden:
Phosphatidinsäure
Phosphatidylethanolemine
Phosphatidylserine
Phosphatidylinositole
Phosphatidylcholine
Cardiolipine
Triglyceride
1,2-Diglyceride
1,3-Diglyceride und
Monoglyceride.

12. Verwendung nach Anspruch 11, wobei die Lipide eines oder mehrere der folgenden Lipide umfassen:
Dioleoylphosphatidylcholin,
Trilinolenin
L-α-Phosphatidinsäure
Dimyristoyl-L-α-phosphatidinsäure
Diheptadecanoyl-D,L-α-phosphatidylcholin
Dimyristoyl-L-α-phosphatidylcholin
Dipentadecanoyl-L-α-phosphatidylcholin
Diheptadecanoyl-D,L-α-phosphatidylcholin
Distearoyl-L-α-phosphatidylethanolamin
Dimyristoyl-D,L-α-phosphatidylethanolamin
Dipalmitoyl-1,2-distearoyl-sn-glycero-3-phosphoethanolamin
L-α-Phosphatidylethanolamin
Diheptadecanoyl-D,L-α-phosphatidyl-L-serin
Dipalmitoyl-N-Palmitoyl-d-sphingosin
N-Palmitoyl-dihydroglucocerebrosid
Trimyristin
Tripentadecanoin
Tripalmitin
1,2-Dipalmitoyl-3-myristoyl-rac-glycerin
1,2-Distearoyl-3-myristoyl-rac-glycerin
1,2-Distearoyl-3-palmitoyl-rac-glycerin und
rac-1,2-Dimyristoyl-3-palmitoylglycerin.

## Revendications

1. Utilisation d'un lipide synthétique, d'un lipide d'origine animale ou d'huile végétale, ledit lipide ayant un squelette glycérol portant au moins un alkyle ou des chaînes acyle, dans laquelle le lipide est un phospholipide, glycolipide ou lipide neutre, et le nombre d'atonies de carbone dans les chaînes hydrocarbonées est entre 10 et 22 inclus, dans la préparation d'une composition pour stimuler et/ou moduler chez un sujet humain ou animal une réponse immunitaire à un anticorps; dans laquelle la composition est adaptée à être donnée à un sujet sous une forme particulaire ou non, par voie parentérale ou transmucosale, et dans laquelle le/s lipide/s présent/s dans la composition est ou/sont **caractérisé**/s par ce qui suit :
a) une ou plusieurs chaînes d'acides gras, chacune ayant une longueur de chaîne de 10 à 22 atomes de carbone, de préférence de 12 à 20, et plus préférablement de 16 à 20, voire 18 à 20 ;
b) où le lipide possède un groupe de tête, le groupe de tête ayant une masse d'au moins 269 unités de masse atomique (AMU) et un volume d'au moins 167 cm⁻³mol⁻¹, et une charge électrique partielle combinée de -1,015eV ou plus fortement négative et préférablement de -1,044eV ou plus négative, et dans lequel le groupe de tête peut avoir un seul centre charge, mais préférablement possède de multiples centres chargés; et
c) où le lipide possède un groupe de tête, le rapport de la charge du groupe de tête par rapport au volume est de préférence -0,004eV/cm⁻³mol⁻¹ ou plus fortement négatif, et plus préférablement -0,006eV/cm⁻³mol⁻¹ ou plus fortement négatif.

2. Utilisation selon la revendication 1, dans laquelle la composition est adaptée pour être administrée par voie transmucosale et comprend des lipides sous forme particulaire.

3. Utilisation selon la revendication 1 ou 2, dans laquelle la composition comprend des lipides transformés en forme particulaire par incorporation dans ou sur des liposomes ou bilosomes ou toute autre matière à taille particulaire appropriée, telle que des particules de silice, de dioxyde de titane, ou de kaolin.

4. Utilisation selon la revendication 3, dans laquelle le lipide ou les lipides sont transformés en un liquide de viscosité faible, comme par chauffage ou dissolution dans des solvants tels que le chloroforme ou le méthanol, ou par transformation en une préparation aqueuse aux moyens d'agent émulsifiant, pour former une émulsion ou un colloïde, et les particules à revêtir sont ensuite dispersées dans le liquide par agitation, par vibration, par sonication ou par tout autre moyen connu de dispersion, le liquide étant ensuite retiré pour laisser un revêtement du lipide ou des lipides sur la surface des particules.

5. Utilisation selon la revendication 4, dans laquelle le liquide est retiré par évaporation de solvants ou du milieu aqueux, par centrifugation (flux séquentiel ou continu) ou filtration, de préférence par filtration à flux croisé.

6. Utilisation selon la revendication 3 ou 4, dans laquelle le lipide est présenté, par exemple par examen microscopique, pour être appliqué aux particules, et un support peut être ajouté selon un volume convenable pour fournir une forme de dosage qui convienne.

7. Utilisation selon l'une des revendications 3 à 5, dans laquelle le lipide est incorporé aux formulations particulaires de poudre humide ou sèche ou de mélanges granuleux, ou alternativement incorporé aux liquides tels que l'eau, l'huile, le lait ou toute boisson.

8. Utilisation selon l'une des revendications 3 à 5, dans laquelle la composition est adaptée pour être administrée sous forme de comprimé, capsule ou pâte, ou incorporée dans la nourriture.

9. Utilisation selon la revendication 5, dans laquelle les particules ont une taille de 0,1 à 100 microns, de préférence de 0,1 à 25 microns et le plus préférablement de 0,2 à 15 ou même de 0,3 à 10 microns, et idéalement de 0,4 à 8 microns ou de 0,5 à 5 microns.

10. Utilisation selon la revendication 1, dans laquelle la composition est adaptée pour être administrée par voie parentérale ou transmucosale sous forme particulaire avec des particules ayant des dimensions de 0,1 à 100 microns, plus spécifiquement de 0,2 à 25 microns, de préférence de 0,2 microns jusqu'à 15 microns ou même de 0,3 jusqu'à seulement 10 microns et idéalement de 0,4 à 8 microns ou de 0,5 à 5 microns.

11. Utilisation selon l'une des revendications 1 à 10, dans laquelle les lipides sont des phospholipides, glycolipides ou lipides neutres, saturés ou non saturés, ramifiés ou non dans leurs chaînes d'acides gras, dans ies catégories de :
Acides phosphatidiques
Phosphatidyléthanolamines
Phosphatidylsérines
Phosphatidylinositols
Phosphatidylcholines
Cardiolipines
Triglycérides
1,2-Diglycerides
1,3-Diglycérides et
Monoglycérides

12. Utilisation selon la revendication 11, dans laquelle les lipides incluent un ou plusieurs de ce qui suit :
Phosphatidylcholine, dioléoyl
Trilinolénine
L-*a*-Phosphatidicacide,
Dimyristoyl L-*a*-Phosphatidicacide,
Diheptadécanoyl DL-*a*-Phosphatidylcholine,
Dimyristoyl L-*a*-Phosphatidylcholine,
Dipentadécanyol L-*a*-Phosphatidylcholine
Diheptadécanoyl DL-*a*-Phosphatidylcholine,
Distéaroyl L-*a*-Phosphatidyléthanolamine,
Dimyristoyl DL-*a*-Phosphatidyléthanolamine
Dipalmitoyl 1,2-Distéaroyl-sn-glycéro-3-phosphoéthanolamine
L-*a*-Phospharidyéthanolamine
Diheptadécanoyl DL-*a*-Phosphatidyle-L-sérine,
Dipalmitoyl N-Palmitoyl-d-sphingosine
N-Palmitoyl dihydroglucocérébroside
Trimyristine
Tripentadécanoine
Tripalmitine
1,2 Dipalmitoyl-3-myristoyl-rac-glycérol
1,2 Distéaroyl-3-myristoyl-rac-glycérol
1,2 Distéaroyl-3-palmitoyl-rac-glycérol et
Rac-1,2-Dimyristoyl-3-palmitoylglycérol
